# FASCICULE DE BREVET EUROPEEN

(11) **EP 0 620 273 B1**
(45) Date de publication et mention de la délivrance du brevet: **12.01.2000**
(21) Numéro de dépôt: 94400590.9
(22) Date de dépôt: 17.03.1994
(51) Int. Cl.: C12M 1/107, C12P 5/00, C02F 11/04

(54) **Procédé et installation de fermentation anaérobie de matières organiques**
Verfahren und Vorrichtung für anaerobe Fermentation von organischen Materialien
Process and system for an aerobic fermentation of organic materials

(30) Priorité: 19.03.1993 FR 9303225
(43) Date de publication de la demande: 19.10.1994
(73) Titulaire: L. & C. Steinmüller GmbH, 51643 Gummersbach (DE)
(72) Inventeur: Cayrol, Francois, F-34000 Montpellier (FR); Fichet, Georges, F-92160 Antony (FR); Lotti, Jean Pierre, F-34130 Valergues (FR); Saint-Joly, Claude, F-34160 Sussargues (FR)
(74) Mandataire: Beauchamps, Georges

(56) Documents cités:
- EP-A- 0 154 334
- FR-A- 996 195
- GB-A- 2 230 004
- US-A- 2 029 702

## Description

La présente invention a essentiellement pour objet un procédé de fermentation anaérobie de matières organiques provenant par exemple de déchets urbains tels que des ordures ménagères.

Elle vise également une installation comportant application de ce procédé.

On connaît déjà des procédés de fermentation anaérobie dans lesquels des matières organiques diluées sont introduites dans plusieurs fermenteurs en parallèle, une partie au moins des matières fermentées étant extraite des fermenteurs et recyclée vers l'amont de ceux-ci où lesdites matières sont mélangées avec les matières diluées que l'on introduit dans lesdits fermenteurs.

Toutefois, ces procédés et installations tels que décrits par exemple dans le document GB-A-2.230.004, présentent un certain nombre d'inconvénients, parmi lesquels il faut citer :
- la difficulté à gérer les fortes variations dans le temps des quantités et de la qualité des matières organiques arrivant à l'installation ;
- la difficulté à gérer les problèmes d'écoulement dans les fermenteurs surtout lorsque ceux-ci présentent un volume important. En effet, dans les fermenteurs de gros volume, de nombreux phénomènes rhéologiques interviennent, tant au niveau de l'introduction des matières que de leur transit dans les fermenteurs, de leur extraction, et de leur écoulement dans les canalisations d'introduction et d'extraction ;
- la difficulté de la conduite des fermenteurs en parallèle, car ceux-ci fonctionnent en quelque sorte indépendamment l'un de l'autre, notamment au niveau du recyclage, de sorte que l'installation nécessite une surveillance constante et indépendante de chaque fermenteur, et un ajustement permanent des paramètres de conduite de chaque fermenteur.
- le coût de main d'oeuvre très important résultant du suivi biologique complet de chaque fermenteur. A cet égard, on observera que l'hétérogénéité des matières à fermenter entraîne une dispersion importante dans la qualité des produits extraits, ce qui complique les traitements à effectuer en aval de l'installation.
- la fragilité de l'ensemble de l'installation tenant au fait que l'hétérogénéité des matières en amont et donc en aval des fermenteurs est aléatoire, ce qui, comme on le comprend, est nuisible tant à la stabilité biologique des fermenteurs qu'à la régularité du fonctionnement général de l'usine de traitement.

La présente invention a pour but de remédier notamment aux inconvénients ci-dessus en proposant un procédé et une installation dont la gestion technique et biologique est nettement simplifiée, dont la régularité d'obtention et la qualité des sous-produits est améliorée, et dont la maîtrise d'écoulement des matières dans les fermenteurs et canalisations est avantageusement assurée.

A cet effet, l'invention a pour objet un procédé de fermentation anaérobie de matières organiques consistant à diluer ces matières, à les introduire dans au moins deux fermenteurs en parallèle, et à recycler au moins une partie des matières fermentées sortant des fermenteurs vers l'amont de ces fermenteurs, où lesdites matières fermentées recyclées sont mélangées avec les matières diluées, caractérisé en ce qu'au moins une partie des matières sortant du premier fermenteur est injectée à l'amont du deuxième fermenteur, tandis qu'au moins une partie des matières fermentées sortant de ce second fermenteur est injectée vers l'amont du premier fermenteur.

Le transfert par croisement entre chaque fermenteur des matières fermentées extraites tout en alimentant en matières organiques ces deux fermenteurs, procure de nombreux avantages qui seront expliqués en détail plus loin.

Selon une autre caractéristique de ce procédé, une partie des matières fermentées sortant du deuxième fermenteur est aussi injectée à l'amont d'un troisième fermenteur, tandis qu'au moins une partie des matières sortant de ce troisième fermenteur est injectée soit à l'amont du premier fermenteur, soit à l'amont du second fermenteur.

On précisera encore ici qu'une partie des matières fermentées sortant d' au moins l'un des fermenteurs précités est déshydratée et le jus obtenu est recyclé vers un mélangeur situé en amont des fermenteurs pour réaliser la dilution des matières à introduire dans le digesteur.

Ce procédé est encore caractérisé en ce qu'on régule le débit des matières sortant d'au moins l'un des fermenteurs et injectées à l'amont d'un autre fermenteur par au moins un régulateur asservissant deux pompes, vannes ou analogues, situées respectivement en amont et en aval de chaque fermenteur de façon à obtenir une viscosité donnée des matières passant dans les fermenteurs.

La présente invention vise également une installation pour la mise en oeuvre du procédé répondant aux caractéristiques ci-dessus, comprenant un mélangeur recevant des déchets organiques mélangés avec un diluant provenant d'un moyen de déshydratation d'une partie de la matière fermentée dans au moins deux fermenteurs, caractérisée par au moins un premier conduit reliant la sortie d'un premier fermenteur à l'amont d'un second fermenteur et par au moins un deuxième conduit reliant la sortie du deuxième fermenteur à l'amont du premier fermenteur.

Cette installation est encore caractérisée en ce que l'aval du deuxième fermenteur est en outre relié à l'amont d'un troisième fermenteur dont l'aval est relié soit à l'amont du deuxième fermenteur, soit à l'amont du premier fermenteur.

On précisera encore ici que les conduits précités sont raccordés à une pompe à l'amont de chaque fermenteur.

Suivant une autre caractéristique de cette installation, en aval d'au moins l'un des fermenteurs est prévu un moyen de déshydratation et au moins un réservoir-tampon prévu sur un conduit reliant le moyen de déshydratation au mélangeur.

Selon encore une autre caractéristique de cette installation, un régulateur de viscosité est monté entre les pompes amont et aval d'un ou de chaque fermenteur.

Il faut encore préciser ici que les pompes amont et/ou aval de chaque fermenteur peuvent constituer une seule pompe amont et/ou une seule pompe aval à laquelle sont associées des vannes ou analogues.

Cette installation peut en outre comporter un régulateur de température relié entre la sortie de la pompe amont d'au moins l'un des fermenteurs et une arrivée de vapeur au mélangeur.

Le croisement du flux extrait d'un fermenteur et injecté à l'amont d'un autre fermenteur, ainsi que la régulation de la viscosité et des températures permettront un fonctionnement optimal de l'installation, comme on l'expliquera en détail plus loin.

D'autres avantages et caractéristiques de l'invention apparaîtront mieux dans la description détaillée qui suit et se réfère aux dessins annexés, donnés uniquement à titre d'exemple, et dans lesquels.

La figure 1 est un schéma bloc d'une installation conforme aux principes de l'invention.

Les figures 2, 3 et 4 sont également des schémas blocs illustrant respectivement trois autres modes de réalisation de cette installation.

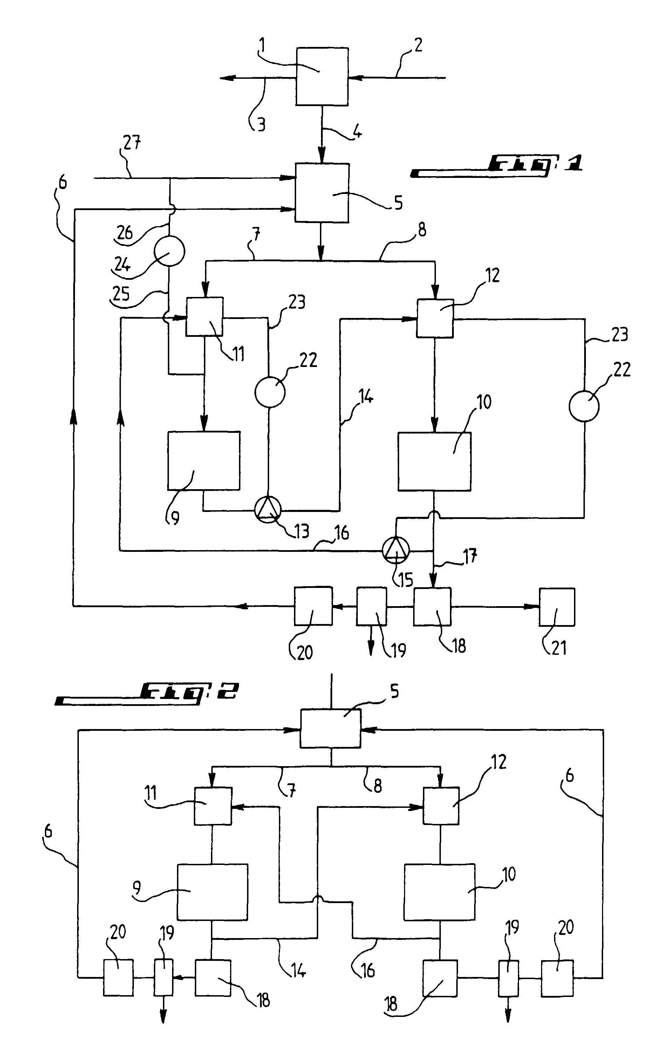

En se reportant à la figure 1, et suivant un exemple de réalisation, on voit une installation de fermentation anaérobie de déchets urbains, tels que des ordures ménagères, arrivant en 2 dans un atelier de tri 1 qui comporte au moins une sortie 3 de matières refusées et une sortie 4 de matières à fermenter parvenant à un mélangeur 5 où elles sont diluées par du jus provenant d'une conduite 6 reliée à l'aval de l'installation, comme on le décrira plus loin.

Les matières diluées alimentent par des conduites 7, 8 deux fermenteurs ou digesteurs repérés respectivement en 9 et 10, et cela respectivement par l'intermédiaire de deux pompes 11, 12.

Conformément à l'invention, la sortie du premier fermenteur 9 est reliée via une pompe 13 par un conduit 14 à la pompe 12 en amont du deuxième fermenteur 10. De la même manière, et cela de façon croisée, la sortie du deuxième fermenteur 10 est raccordée par un conduit 16 et via une pompe 15 à la pompe 11 en amont du premier fermenteur 9.

La conduite 14 fait passer l'intégralité de la matière extraite du premier fermenteur 9 dans le deuxième fermenteur 10 via la pompe 12, tandis que la deuxième conduite 16 ne fait passer qu'une partie de la matière extraite du deuxième fermenteur 10 vers le premier fermenteur 9 via la pompe 11. L'autre partie extraite du deuxième fermenteur 10 est acheminée par une conduite 17 dans un système de déshydratation 18 raccordé par la conduite 6, précédemment mentionnée, au mélangeur 5, ladite conduite 6 transportant le jus issu du moyen de déshydratation 18 qui peut être une presse par exemple.

Sur la conduite 6 est prévu un système 19 de post-traitement des jus, si nécessaire, et un réservoir-tampon 20 permettant un stockage intermédiaire des jus servant à la dilution. On a montré en 21 un atelier de post-traitement du produit solide déshydraté en 18.

L'installation qui vient d'être décrite comporte avantageusement une régulation notamment de la viscosité et de la température qui sera décrite ci-après.

On voit en 22 deux régulateurs de viscosité raccordés par une boucle 23 aux pompes et aux fermenteurs comme suit : l'un des régulateurs 22 est relié à la pompe 11 et à la pompe 13 sur la sortie du premier fermenteur 9, et l'autre régulateur 22 est relié à la pompe 12 et à la pompe 15 sur le conduit 16 de sortie du deuxième fermenteur 10.

Ces régulateurs asserviront avantageusement le débit des pompes 13, 15 à une mesure directe ou indirecte de la viscosité effectuée au niveau des pompes 11, 12. Il est à noter qu'on pourrait parfaitement, sans sortir du cadre de l'invention, utiliser des vannes (non représentées), à la place des pompes 13 et 15, dans le cas où les fermenteurs 9, 10 alimentent par gravité respectivement les pompes 12 et 11.

On a montré schématiquement en 24 sur la figure 1 un régulateur de température relié par des conduites 25, 26 respectivement à la sortie de la pompe 11 ou 12 (bien que cela ne soit pas représenté pour la pompe 12), et à une entrée de vapeur 27 dans le mélangeur 5.

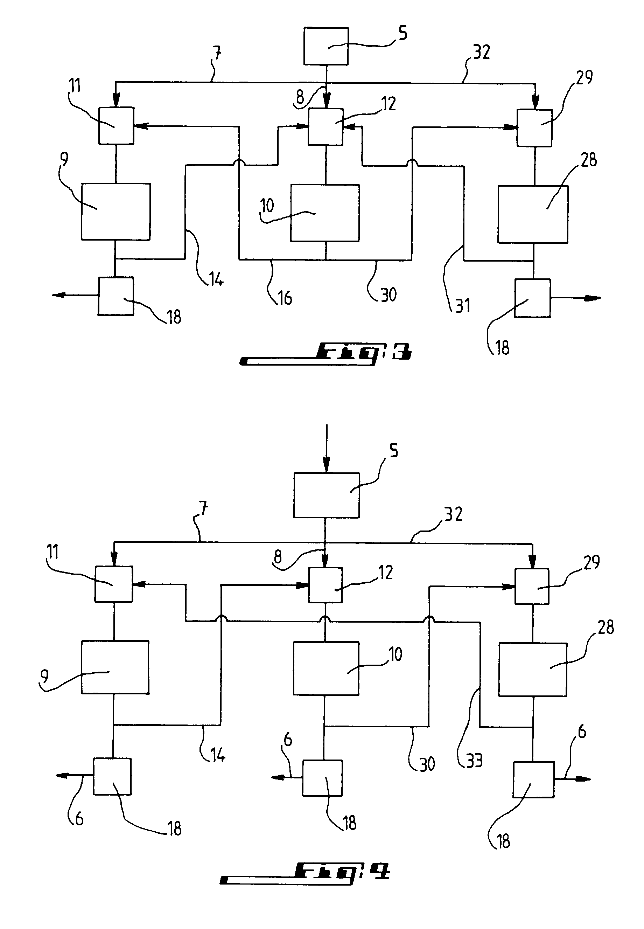

Le mode de réalisation illustré par la figure 2 est identique à celui de la figure 1 décrit précédemment, au regard du croisement des flux 14, 16 sortant d'un fermenteur et injectés vers l'amont de l'autre fermenteur.

La seule différence ici est qu'à la sortie de chaque fermenteur 9, 10 est prévu un système de déshydratation 18, qui effectue la déshydratation d'une partie de la matière sortant de chaque fermenteur ou digesteur, le jus étant recyclé à partir de chaque système de déshydratation 18 par un conduit 6 vers le mélangeur 5. On observera ici que, sans sortir du cadre de l'invention, les systèmes 18, 19 et 20 peuvent former un seul système commun aux deux fermenteurs 9 et 10.

Dans le mode de réalisation illustré par la figure 3, aux deux fermenteurs 9 et 10 est associé un troisième fermenteur ou digesteur 28 en amont duquel est prévue une pompe 29 reliée au mélangeur 5.

Une partie de la matière extraite du deuxième fermenteur 10 est acheminée via une conduite 30 à l'amont du troisième fermenteur 28, et cela en étant raccordée à la pompe 29. La sortie de la matière extraite du troisième fermenteur 28 est reliée via un conduit 31 à l'amont du deuxième fermenteur 10 via la pompe 12. On réalise donc ici un croisement des flux entre deuxième et troisième fermenteurs, lequel croisement est complémentaire de celui entre premier fermenteur 9 et deuxième fermenteur 10.

On remarquera ici qu'un système de déshydratation tel que 18 n'est prévu qu'à la sortie du premier fermenteur 9 et du troisième fermenteur 28. Bien entendu, le troisième fermenteur 28 sera raccordé via la pompe 29 à la sortie du mélangeur 5 par une conduite repérée en 32.

La variante illustrée dans la figure 4 s'apparente à celle de la figure 3. La sortie du premier fermenteur 9 est toujours reliée par la conduite 14 à l'amont du deuxième fermenteur 10, la sortie 30 du deuxième fermenteur 10 est toujours raccordée à l'amont du troisième fermenteur 28, mais, ici, la sortie de la matière extraite du troisième fermenteur 28 est reliée par une canalisation 33 à l'amont du premier fermenteur 9 via la pompe 11. On observera encore que l'on a prévu ici, à la sortie de chaque fermenteur 9, 10, 28 un système de déshydratation 18 produisant un jus acheminé par la conduite 6 au mélangeur 5 pour y réaliser une dilution avec les matières organiques fraîches alimentation l'installation. A la place des trois systèmes 18 complétés par les systèmes 19 et 20 (non représentés sur les figures 3 et 4), on peut parfaitement prévoir un ensemble qui serait commun aux trois fermenteurs, comme cela a été décrit à propos de la réalisation de la figure 2.

Le fonctionnement des installations des figures 1 à 4 se déduit immédiatement de la description qui vient d'en être faite, mais pour une meilleure compréhension de l'invention, on en donnera ci-après quelques détails en insistant sur les effets et avantages obtenus par rapport aux procédés et installations classiques, c'est-à-dire comprenant une pluralité de fermenteurs fonctionnant, biologiquement parlant, en parallèle et constitués par des cuves de fermentation ayant un volume relativement important, par exemple de plusieurs centaines de mètres cubes.

Tout d'abord, si l'on compare le rapport tonnage déchets traités/tonnage déchets collectés et amenés à l'usine, on constate que ce rapport est de 100% pour le procédé et l'installation de l'invention, alors qu'auparavant, il était de 73,5%.

En d'autres termes, la disponibilité générale de l'installation est bien meilleure qu'auparavant, ce qui permet une gestion très simplifiée en raison notamment des effets induits à tous les niveaux par le croisement des flux recirculés et la régulation de la viscosité et de la température.

Pour ce qui concerne les variations de la charge admissible, on a constaté que, d'un jour à l'autre, la variation peut être de ± 20% pour l'installation de l'invention, alors qu'elle était de ± 10% pour les installations traditionnelles.

Cette variation, d'une semaine à l'autre peut être de 5 à 30% pour une installation selon l'invention, alors qu'elle était de 5 à 10% pour une installation classique.

Ces résultats sont la preuve de la souplesse du procédé et de l'installation de l'invention, et cela tant pour des variations de quantité que pour des variations de qualité des matières organiques à traiter.

En bref, tout en alimentant quotidiennement en matières fraîches chacun des fermenteurs, on arrive à un comportement équivalent à un seul fermenteur de grande taille sans les inconvénients de ce fermenteur unique résultant des phénomènes rhéologiques complexes des matières régissant les conditions d'écoulement, d'agitation etc..., et cela en réalisant, comme expliqué plus haut, le croisement, selon diverses modalités, des matières extraites de chacun des fermenteurs.

Pour ce qui concerne le moyen de pressage procurant la déshydratation des matières méthanisées, on a constaté l'amélioration des performances (observées sur plusieurs mois) à tous points de vue entre une installation classique et une installation selon l'invention.
- débit moyen journalier légèrement amélioré, passant de 16,5 à 18,6 t/h,
- débit minimum journalier fortement relevé, passant de 6,9 à 12,8 t/h,
- plage de variation de débit fortement réduite, avec un écart-type passant de 3,5 à 2,5 t/h,
- plage de variation de qualité du produit (teneur en matières sèches finales) fortement réduite, avec un écart-type passant de 4,4 à 3,5% MS.

Enfin, pour ce qui est de la qualité du biogaz produit, on fera les observations suivantes.

Pour une teneur en méthane produit égale dans les deux cas (tant pour une installation classique que pour une installation selon l'invention), on obtient une régularité de la qualité beaucoup plus grande. En effet, on a trouvé que pour une teneur hebdomadaire moyenne en CH₄ inchangée (54%), on limitait fortement les variations instantanées passant d'une fourchette 40-68% pour une installation classique, à une fourchette 45-63% pour une installation selon l'invention.

On a donc réalisé suivant l'invention une installation remarquablement stabilisée sur le plan biologique, pouvant recevoir, sans inconvénients, de fortes variations qualitatives et quantitatives de charge, conduisant à une très grande régularité de la qualité des sous-produits gazeux, solides et liquides issus de la fermentation, et conduisant à une simplification importante du suivi biologique. En outre, grâce à ce procédé et à cette installation, on obtient un contrôle très efficace des conditions d'écoulement, d'agitation et de transfert des matières, et cela à tous les niveaux dudit procédé ou de ladite installation. Enfin, grâce à l'invention, on peut mettre en oeuvre des fermenteurs de taille raisonnable permettant de garantir une maîtrise des phénomènes rhéologiques tout en disposant de tous les avantages d'une grande capacité de digestion.

## Revendications

1. Procédé de fermentation anaérobie de matières organiques consistant à diluer ces matières, à les introduire dans au moins deux fermenteurs (9, 10) en parallèle et à recycler au moins une partie des matières fermentées sortant des fermenteurs vers l'amont de ces fermenteurs, où les matières fermentées recyclées sont mélangées avec les matières diluées, caractérisé en ce qu'au moins une partie des matières sortant du premier fermenteur (9) est injectée à l'amont du deuxième fermenteur (10), tandis qu'au moins une partie des matières fermentées sortant du second fermenteur (10) est injectée vers l'amont du premier fermenteur (9).

2. Procédé selon la revendication 1, caractérisé en ce qu'au moins une partie des matières fermentées sortant du deuxième fermenteur (10) est aussi injectée à l'amont d'un troisième fermenteur (28), tandis qu'au moins une partie des matières sortant de ce troisième fermenteur (28) est injectée soit à l'amont du premier fermenteur (9) soit à l'amont du second fermenteur (10).

3. Procédé selon la revendication 1 ou 2, caractérisé en ce qu'une partie des matières fermentées sortant d'au moins l'un des fermenteurs (9, 10, 28) précités est déshydratée et le jus obtenu est recyclé vers un mélangeur (5) situé en amont des fermenteurs pour réaliser la dilution des matières à introduire dans les fermenteurs.

4. Procédé selon l'une des revendications 1 à 3, caractérisé en ce qu'on régule le débit des matières sortant d'au moins l'un des fermenteurs et injectées à l'amont d'un autre fermenteur, par au moins un régulateur (22) asservissant deux pompes (13, 15, 11, 12), vannes ou analogues situées respectivement en amont et en aval de chaque fermenteur de façon à obtenir une viscosité donnée des matières passant dans les fermenteurs.

5. Installation pour la mise en oeuvre du procédé selon l'une des revendications 1 à 4, comprenant un mélangeur (5) recevant des déchet organiques mélangés avec un diluant provenant d'un moyen de déshydratation (18) d'une partie de la matière fermentée dans au moins deux fermenteurs (9, 10), caractérisée par au moins un premier conduit (14) reliant la sortie d'un premier fermenteur (9) à l'amont d'un second fermenteur (10) et par au moins un deuxième conduit (16) reliant la sortie du deuxième fermenteur (10) à l'amont du premier fermenteur (9).

6. Installation selon la revendication 5, caractérisée en ce que l'aval du deuxième fermenteur (10) est en outre relié à l'amont d'un troisième fermenteur (28) dont l'aval est relié soit à l'amont du deuxième fermenteur (10), soit à l'amont du premier fermenteur (9).

7. Installation selon la revendication 5 ou 6, caractérisée en ce que les conduits précités sont reliés à une pompe (11, 12, 29) à l'amont de chaque ermenteur.

8. Installation selon l'une des revendications 5 à 7, caractérisée en ce qu'en aval d'au moins l'un des fermenteurs (9, 10, 28) est prévu un moyen de déshydratation (18) et au moins un réservoir-tampon (20) prévu sur une conduite (6) reliant le moyen de déshydratation (18) au mélangeur (5).

9. Installation selon l'une des revendications 5 à 8, caractérisée en ce qu'il comprend un régulateur de viscosité (22) monté entre les pompes amont (11,12,29) et aval (13,15) d'un ou de chaque fermenteur (9,10,28).

10. Installation selon l'une des revendications précédentes, caractérisée en ce que les pompes précitées amont et/ou aval de chaque fermenteur peuvent constituer respectivement une seule pompe amont et/ou une seule pompe aval à laquelle sont associées des vannes, ou analogues.

11. Installation selon l'une des revendications précédentes, caractérisée par un régulateur de température (24) relié entre la sortie de la pompe amont (11,12,29) d'au moins l'un des fermenteurs et une arrivée de vapeur (27) au mélangeur (5).

## Patentansprüche

1. Verfahren für anaerobe Fermentation von organischen Materialien, darin bestehend, daß diese Materialien aufgelöst, in wenigstens zwei parallele Fermenter (9, 10) eingeführt werden und daß wenigsten ein Teil der oberhalb dieser Fermenter, wo die recycleten fermentierten Materialien mit den verdünnten Materialien vermischt werden, austretenden fermentierten Materialien recycled werden, dadurch gekennzeichnet, daß wenigstens ein Teil der aus dem ersten Fermenter (9) austretenden Materialien oberhalb des zweiten Fermenters (10) zugeführt wird, während wenigstens ein Teil der aus dem zweiten Fermenter (10) austretenden fermentierten Materialien oberhalb des ersten Fermenters (9) zugeführt wird.

2. Verfahren gemäß Anspruch 1, dadurch gekennzeichnete, daß wenigstens ein Teil der aus dem zweiten Fermenter (10) austretenden fermentierten Materialien auch oberhalb eines dritten Fermenters (28) zugeführt wird, während wenigstens ein Teil der aus diesen dritten Fermenter (28) austretende Materialien entweder oberhalb des ersten Fermenters (9) zugeführt wird oder oberhalb des zweiten Fermenters (10).

3. Verfahren gemäß Anspruch 1 oder 2, dadurch gekennzeichnet, daß ein Teil der wenigstens aus einem der besagten Fermenter (9, 10, 28) austretenden fermentierten Materialien entwässert wird und die gewonnene Flüssigkeit in einen Mischer (5) oberhalb der Fermenter recycled wird, um die Verdünnung der in die Fermenter eingeführten Materialien zu realisieren.

4. Verfahren gemäß Anspruch 1 bis 3, dadurch gekennzeichnet, daß man den Durchfluß der aus wenigstens einem der Fermenter austretenden und oberhalb eines anderen Fermenters zugeführten Materialien durch wenigstens einen Regulator (22) regelt, der zwei jeweils derart oberhalb und unterhalb jedes Fermenters gelegenen Pumpen (13, 15, 11, 12), Ventile oder analoge regelt, daß eine bestimmte Viskosität der durch die Fermenter durchfließende Materialien gegeben ist.

5. Vorrichtung für die Durchführung des Verfahrens gemäß Anspruch 1 bis 4, bestehend aus einem Mischer (5), der die organischen Abfälle aufnimmt, die mit einem Verdünner vermischt sind, der aus einem Entwässerungsmittel (18) eines Teils der in wenigstens zwei Fermentern (9, 10) fermentierten Materialien stammt, gekennzeichnet durch wenigstens eine erste Leitung (14), die an den Austritt eines ersten Fermenters (9) oberhalb des zweiten Fermenters (10) angeschlossen ist und durch wenigstens eine zweite Leitung (16), die an den Ausgang des zweiten Fermenters (10) oberhalb des ersten Fermenters (9) angeschlossen ist.

6. Vorrichtung gemäß Anspruch 5, dadurch gekennzeichnet, daß der obere Teil des zweiten Fermenters (10) außerdem an den unteren Teil des dritten Fermenters (28) angeschlossen ist, dessen unterer Teil entweder an den oberen Teil des zweiten Fermenters (10) oder an den oberen Teil des ersten Fermenters (9) angeschlossen ist.

7. Vorrichtung gemäß Anspruch 5 oder 6, dadurch gekennzeichnet, daß die besagten Leitungen an eine Pumpe (11, 12, 29) oberhalb jedes Fermenters angeschlossen sind.

8. Vorrichtung gemäß Anspruch 5 bis 7, dadurch gekennzeichnet, daß unterhalb wenigstens eines des Fermenters (9, 10, 28) ein Entwässerungsmittel (18) vorgesehen ist und wenigstens ein Auffangbehälter (20) auf einer Leitung (6) vorgesehen ist, die die Entwässerungsmittel (18) mit dem Mischer (5) verbinden.

9. Vorrichtung gemäß Anspruch 5 bis 8, dadurch gekennzeichnet, daß sie einen zwischen den Pumpen oberhalb (11, 12, 29) und unterhalb (13, 15) eines oder jedes Fermenters (9, 10, 28) eingebauten Viskositätsregulator (22) umfaßt.

10. Vorrichtung gemäß einem der vorherigen Ansprüche, dadurch gekennzeichnet, daß die besagten Pumpen oberhalb und / oder unterhalb jedes Fermenters jeweils eine einzige Pumpe oberhalb und / oder eine einzige Pumpe unterhalb sein können, der Ventile oder ähnliches zugeordnet sind.

11. Vorrichtung gemäß einem der vorigen Ansprüche, gekennzeichnet durch einen Temperaturregler (24) , der zwischen dem Austritt der Pumpe oberhalb (11, 12, 29) wenigstens eines der Fermenter und einem Dampfeintritt (27) an den Mischer (5) angeschlossen ist.

## Claims

1. Process for aerobic fermentation of organic materials consisting of diluting these materials, introducing them into at least two fermentors (9, 10) in parallel and of recycling at least one portion of the fermented materials coming out the fermentors towards the upstream portion of these fermentors where the recycled fermented materials are mixed with the diluted materials, characterised in that at least one portion of the materials coming out of the first fermentor (9) is injected towards the upstream portion of the second fermentor (10), whereas at least one portion of the fermented materials coming out of the second fermentor (10) is injected towards the upstream portion of the first fermentor (9).

2. Process according to claim 1, characterised in that at least one portion of the fermented materials coming out of the second fermentor (10) is also injected to the upstream portion of a third fermentor (28), whereas at least one portion of the materials coming out of this third fermentor (28) is injected either to the upstream portion of the first fermentor (9) or to the upstream portion of the second fermentor (10).

3. Process according to claim 1 or 2, characterised in that one portion of the fermented materials coming out of at least one of said fermentors (9, 10, 28) is dehydrated and the juice obtained is recycled towards a mixer (5) situated upstream of the fermentors so as to carry out dilution of the materials to be introduced in the fermentors.

4. Process according to one of claims 1 to 3, characterised in that the flow of the materials coming out of at least one of the fermentors is regulated and injected to the upstream portion of another fermentor by at least one regulator (22) controlling two pumps (13, 15, 11, 12), valves or similar elements situated respectively upstream and downstream of each fermentor so as to obtain a given viscosity of the materials passing inside the fermentors.

5. System for implementing the process according to one of claims 1 to 4 and including a mixer (5) receiving organic waste mixed with a diluting agent originating from a device (18) for dehydrating a portion of the fermented material in at least two fermentors (9, 10), characterised by at least one first pipe (14) connecting the outlet of a first fermentor (9) to the upstream portion of a second fermentor (10) and by at least a second pipe (16) connecting the outlet of the second fermentor (10) to the upstream portion of the first fermentor (9).

6. System according to claim 5, characterised in that the downstream portion of the second fermentor (10) is further connected to the upstream portion of a third fermentor (28) whose downstream portion is connected either to the upstream portion of the second fermentor (10) or to the upstream portion of the first fermentor (9).

7. System according to claim 5 or 6, characterised in that said pipes are connected to a pump (11, 12, 29) at the upstream portion of each fermentor.

8. System according to one of claims 5 to 7, characterised in that downstream of at least one of the fermentors (9, 10, 28) is provided with a dehydration device (18) and at least one stopper tank (20) provided on a pipe (6) connecting the dehydration device (18) to the mixer (5).

9. System according to one of claims 5 to 8, characterised in that it includes a viscosity regulator (22) mounted between the upstream (11, 12, 29) and downstream (13, 15) pumps of one or each fermentor (9, 10, 28).

10. System according to one of the preceding claims, characterised in that said upstream and/or downstream pumps of each fermentor can respectively constitute a single upstream and/or downstream pump with which valves or similar elements are associated.

11. System according to one of the preceding claims, characterised by a temperature regulator (24) connected between the outlet of the upstream pump (11, 12, 29) of at least one of the fermentors and a vapour intake (27) to the mixer (5).
